(19) Europäisches Patentamt
European Patent Office
Office européen des brevets

(11) **EP 3 416 291 A2**

(12) **EUROPEAN PATENT APPLICATION**

(43) Date of publication:
**19.12.2018   Bulletin 2018/51**

(51) Int Cl.:
**H03M 1/12** *(2006.01)*

(21) Application number: **18183261.9**

(22) Date of filing: **12.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.05.2017   JP 2017103835**
**15.09.2017   JP 2017177551**

(71) Applicant: **Renesas Electronics Corporation**
**135-0061 Tokyo (JP)**

(72) Inventors:
• **Tadashi, Imokawa**
**Tokyo 135-0061 (JP)**

• **Norio, Okada**
**Tokyo 135-0061 (JP)**

(74) Representative: **Glawe, Delfs, Moll**
**Partnerschaft mbB von**
**Patent- und Rechtsanwälten**
**Postfach 13 03 91**
**20103 Hamburg (DE)**

Remarks:
A request for re-establishment of rights in respect of the twelve-month period from the date of filing of the first application has been granted (Art.87(1) and Art.122 EPC).

(54) **DETECTION SYSTEM, SENSOR AND MICROCOMPUTER**

(57)    The present disclosure is aimed at correcting a frequency of a clock signal of a sensor based on a clock signal input from a microcomputer. A detection system includes a sensor and a microcomputer. The sensor is configured to be able to output sampling data generated by performing analog/digital conversion on an analog signal sampled based on a clock signal. The microcomputer generates a clock signal and outputs the clock signal to the sensor, and reads out the sampling data from the sensor. The sensor corrects the frequency of the clock signal based on the clock signal.

Fig. 4

EP 3 416 291 A2

**Description**

BACKGROUND

The present disclosure relates to a detection system, a sensor, and a microcomputer.

In recent years, a detection system that acquires data from various sensors and processes the acquired data has been used. One example that has been proposed is a sensor system that processes data acquired by a plurality of sensors (Japanese Unexamined Patent Application Publication No. 2015-228171).

In this system, data communication is performed among a controller, a first sensor, and a second sensor. In this example, a first input terminal of the controller and an input/output terminal of the first sensor are connected to each other. A second input terminal of the controller and an input/output terminal of the second sensor are connected to each other. The input/output terminal of the first sensor and the input/output terminal of the second sensor are connected to each other. The second sensor receives a first sensor signal, and outputs a second sensor signal including a second synchronization signal and a second sensor data which is based on the second synchronization signal in serial in response to the input first sensor signal. Accordingly, in this system, data output from at least two or more sensors becomes data output from the sensors obtained in one period, and this can be achieved by a simple sensor system and simple sensors.

SUMMARY

In the aforementioned configuration, however, while the timings when the data are output from the sensors can be synchronized with one another, it is possible that the timings when the data are sampled may not be the same. In this case, after the sensor converts an analog signal indicating the result of the detection into a digital signal, the sensor outputs the data. In this case, analog/digital conversion is performed in accordance with a clock signal, which serves as a reference of the sampling timing. Therefore, in order to synchronize the timings when the data are sampled at the plurality of sensors with a high accuracy, it is required to maintain the accuracy of the frequencies of the clock signals used in the respective sensors. However, each of the sensors does not typically include a crystal oscillation circuit or the like with a high accuracy since it will lead to an increase in the power consumption and an increase in cost. Therefore, a simple oscillation circuit such as a ring oscillator is typically used. However, there is a problem in the simple oscillation circuit that the frequency tends to fluctuate.

The other problems and novel characteristics will be made apparent from the description of the specification and the attached drawings.

According to one embodiment, a detection system includes: a sensor configured to be able to output sampling data generated by performing analog/digital conversion on an analog signal sampled based on a first clock signal; and a microcomputer configured to generate a second clock signal and output the second clock signal to the sensor, and read out the sampling data from the sensor, in which the sensor corrects a frequency of the first clock signal based on the second clock signal.

According to one embodiment, a sensor is configured to be able to output sampling data generated by performing analog/digital conversion on an analog signal sampled based on a first clock signal, in which a frequency of the first clock signal is corrected based on a second clock signal generated by a microcomputer, and the sampling data is read out by the microcomputer.

According to one embodiment, a microcomputer is configured to generate a second clock signal and output the second clock signal to a sensor configured to be able to output sampling data generated by performing analog/digital conversion on an analog signal sampled based on a first clock signal, the microcomputer being further configured to be able to read out the sampling data from the sensor, in which a frequency of the first clock signal is corrected by the sensor based on the second clock signal.

According to one embodiment, it is possible to correct the frequency of the clock signal of the sensor based on the clock signal input from the microcomputer.

BRIEF DESCRIPTION OF THE DRAWINGS

The above and other aspects, advantages and features will be more apparent from the following description of certain embodiments taken in conjunction with the accompanying drawings, in which:

Fig. 1 is a diagram schematically showing a basic configuration of a detection system according to a first embodiment;
Fig. 2 is a diagram schematically showing a configuration of the detection system according to the first embodiment;
Fig. 3 is a diagram showing an operation timing of the detection system according to the first embodiment;
Fig. 4 is a diagram showing a configuration of a sensor according to the first embodiment in more detail;
Fig. 5 is a diagram showing a configuration of a frequency/voltage converter;
Fig. 6 is a diagram showing a configuration of a voltage holding circuit;
Fig. 7 is a diagram showing a configuration of an oscillator;
Fig. 8 is a diagram showing a recovery operation when the sensor has unexpectedly entered a sleep mode;
Fig. 9 is a diagram showing a recovery operation when a microcomputer has unexpectedly entered the sleep mode;

Fig. 10 is a diagram schematically showing a configuration of a detection system according to a second embodiment;

Fig. 11 is a diagram schematically showing a configuration of a detection system according to a third embodiment;

Fig. 12 is a diagram showing an operation timing of the detection system according to the third embodiment;

Fig. 13 is a diagram schematically showing a configuration of a detection system according to a fourth embodiment;

Fig. 14 is a diagram schematically showing a configuration of a modified example of the detection system according to the fourth embodiment;

Fig. 15 is a diagram schematically showing a configuration of a detection system according to a fifth embodiment;

Fig. 16 is a diagram schematically showing a configuration of a detection system according to a sixth embodiment; and

Fig. 17 is a diagram schematically showing a configuration of a modified example of the detection system according to the sixth embodiment.

DETAILED DESCRIPTION

Specific embodiments of the present disclosure will be explained hereinafter with reference to the drawings. In the drawings, the same elements are denoted by the same reference signs, and repetitive descriptions will be avoided as necessary for clarity of explanation.

First Embodiment

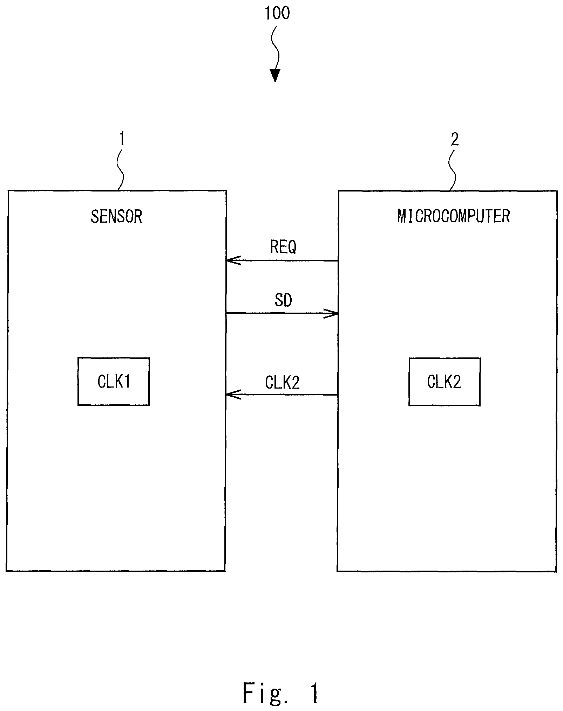

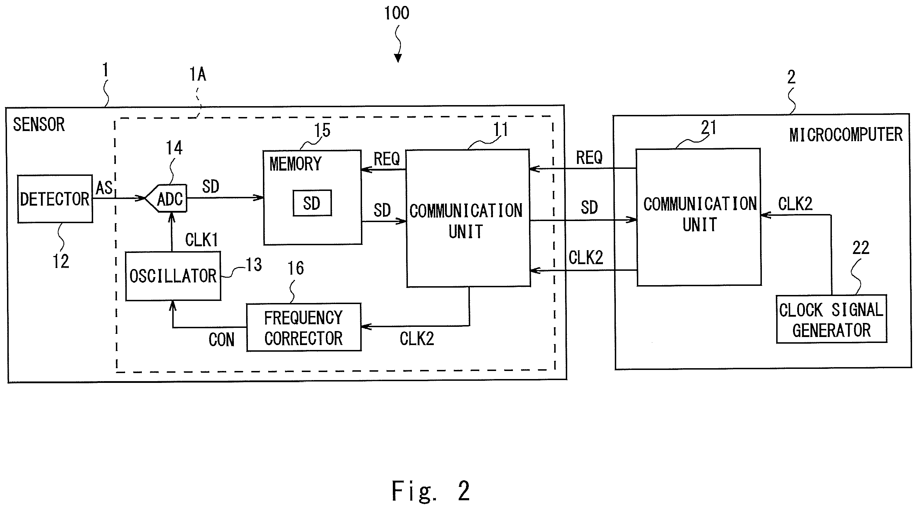

Hereinafter, with reference to the drawings, embodiments of the present disclosure will be explained. Fig. 1 is a diagram schematically showing a basic configuration of a detection system 100 according to a first embodiment. Fig. 2 is a diagram schematically showing a configuration of the detection system 100 according to the first embodiment.

The detection system 100 includes a sensor 1 and a microcomputer 2. The sensor 1 and the microcomputer 2 can perform data communication with each other. The data communication between the sensor 1 and the microcomputer 2 may be performed either by wired communication or wireless communication. The sensor 1 is configured to be able to detect, for example, a physical amount such as an amount of pressure or an amount of acceleration and store the result of the detection in an internal memory. The microcomputer 2 is configured in such a way that it is capable of reading out data indicating the result of the detection from the sensor 1 and controlling operations of the sensor 1.

First, the sensor 1 will be explained. The sensor 1 includes a communication unit 11, a detector 12, an oscillator 13, an analog/digital (A/D) converter 14, a memory 15, and a frequency corrector 16. The communication unit 11, the oscillator 13, the analog/digital (A/D) converter 14, the memory 15, and the frequency corrector 16 compose a signal processor 1A, which is an A/D conversion unit that performs A/D conversion on an analog signal from the detector 12.

The communication unit 11 (this unit is also referred to as a first input/output interface) is an input/output apparatus for performing data communication with the microcomputer 2.

The detector 12 detects a predetermined physical amount such as an amount of pressure or an amount of acceleration and outputs the result of the detection as an analog signal AS.

The oscillator 13 generates a clock signal CLK1 by an oscillation operation and outputs the generated signal to the A/D converter 14. In this embodiment, the oscillator 13 has a relatively simple configuration and is formed of, for example, a ring oscillator. In this case, the oscillator 13 has a characteristic that the oscillation frequency tends to fluctuate due to changes over time and environmental changes. Therefore, in order to maintain the frequency of the clock signal CLK1 at a predetermined value, the oscillator 13 is configured to be able to adjust the frequency of the clock signal CLK1 in accordance with a control signal CON input from the frequency corrector 16.

The A/D converter 14 performs analog/digital conversion (A/D conversion) on the analog signal AS sampled based on the clock signal CLK1 received from the oscillator 13 and outputs the digital signal after the conversion as sampling data SD.

The memory 15 includes a function of storing the sampling data SD sequentially output from the A/D converter 14. The memory 15 outputs the sampling data SD stored therein to the microcomputer 2 via the communication unit 11 in accordance with a data read request REQ from the microcomputer 2. The memory 15 may be, for example, a FIFO (First In, First Out).

The frequency corrector 16 receives a clock signal CLK2 input from the microcomputer 2 via the communication unit 11, and outputs the control signal CON to correct the frequency of the clock signal CLK1 output from the oscillator 13 to the oscillator 13 based on the clock signal CLK2. The details of the operation of correcting the frequency of the clock signal CLK1 will be described later.

Next, the microcomputer 2 will be explained. As described above, the microcomputer 2 is configured to be able to read the sampling data SD from the sensor 1. The microcomputer 2 includes a communication unit 21 and a clock signal generator 22. The microcomputer 2 further includes a CPU and a memory, although they are not shown in the drawings.

The communication unit 21 (this unit is also referred to as a second input/output interface) is an input/output apparatus to perform data communication with the sensor 1.

The clock signal generator 22 includes, for example, an oscillation circuit, and outputs the clock signal CLK2 used for processing of elements provided in the microcomputer 2 based on the oscillation operation. The clock signal CLK2 is output also to the sensor 1 along with, for example, the read request REQ via the communication unit 21. As described above, the clock signal CLK2 that has been input to the sensor 1 is used for the correction processing in the frequency corrector 16.

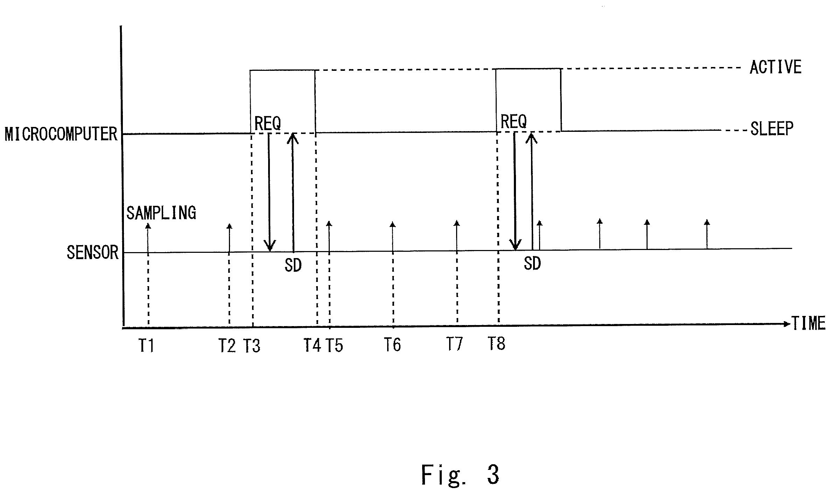

Fig. 3 shows one example of the operation timing. The sensor 1 samples the signal output from the detector 12 (the analog signal AS) at time T1, converts the signal into a digital signal (sampling data SD) by the A/D converter 14, and then holds the converted signal in the memory 15. In a similar way, the sensor 1 samples the signal output from the detector 12 (the analog signal AS) at time T2, converts the signal into a digital signal (sampling data SD) by the A/D converter 14, and then holds the converted signal in the memory 15. Next, after the mode of the microcomputer 2 has changed from the sleep mode to the active mode at time T3, the microcomputer 2 outputs the clock signal CLK2 and the read request REQ to the sensor 1. Upon receiving the read request REQ, the sensor 1 outputs the sampling data SD at times T1 and T2 held in the memory 15 to the microcomputer via the communication unit 11. At time T4, the microcomputer 2 enters the sleep mode. In a similar way, when the microcomputer 2 enters the active mode at time T8, the sampling data SD at times T5, T6, and T7 is output from the sensor 1.

In this embodiment, the clock signal generator 22 includes the oscillation circuit capable of stabilizing the frequency of the clock signal CLK2 with a high accuracy (e.g., a crystal oscillation circuit or an on-chip oscillation circuit adjusted with a high accuracy), and is configured in such a way that it has a frequency stability higher than that of the oscillator 13.

Further, the microcomputer 2 has a function of arranging the plurality of pieces of sampling data SD sequentially read out from the sensor 1 in time series based on the clock signal CLK2 and analyzing these data.

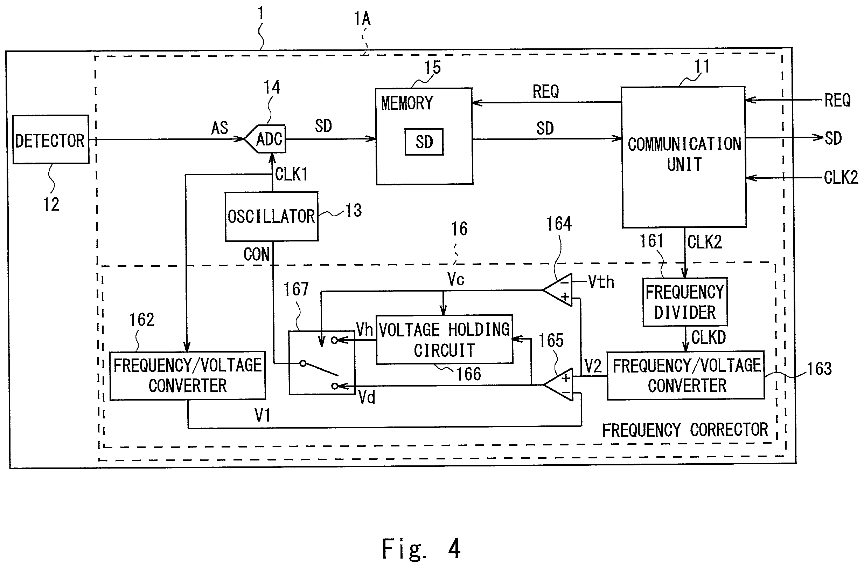

Next, processing for correcting the clock signal CLK1 will be explained. Fig. 4 is a diagram showing the configuration of the sensor 1 according to the first embodiment in further detail. As shown in Fig. 4, the frequency corrector 16 includes a frequency divider 161, a frequency/voltage converter 162, a frequency/voltage converter 163, a comparator 164, a differential amplifier 165, a voltage holding unit (voltage holding circuit) 166, and a switch 167.

The clock signal CLK2 is input to the frequency divider 161 from the microcomputer 2 via the communication unit 11. The frequency divider 161 divides the frequency of the clock signal CLK2 by a predetermined ratio n. That is, the frequency divider 161 outputs, when the frequency of the clock signal CLK2 is represented by f, a frequency-divided signal CLKD having a frequency of f/n.

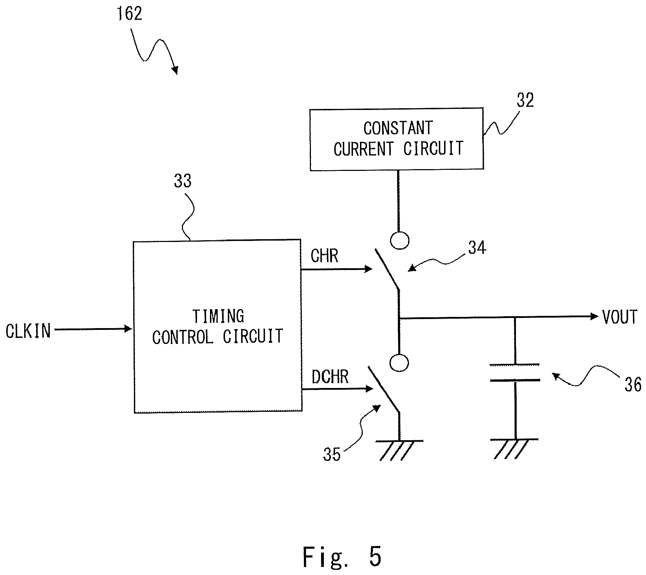

The frequency/voltage converter 162 (this converter is also referred to as a first frequency/voltage converter) converts the clock signal CLK1 output from the oscillator 13 into a voltage signal V1 (this signal is also referred to as a first signal). Fig. 5 shows one example of the configuration of the frequency/voltage converter 162. A clock signal CLKIN (this signal corresponds to CLK1) is input to a timing control circuit 33. The timing control circuit 33 generates a charge signal CHR and a discharge signal DCHR based on the clock signal CLKIN. A switch 34 is provided in such a way that it allows electrical conduction between a constant current circuit 32 and a capacitor 36, and the switch 34 is controlled to be turned on or off by the charge signal CHR. A switch 35 is provided in such a way that it can connect the capacitor 36 and the ground potential, and the switch 35 is controlled to be turned on or off by the discharge signal DCHR. Therefore, in accordance with the clock signal CLKIN, electric charges are charged in the capacitor 36 by the current output from the constant current circuit 32 and the electric charges held in the capacitor 36 are discharged, whereby a voltage VOUT in accordance with the frequency of the clock signal CLKIN (this corresponds to the voltage signal V1) is output.

The frequency/voltage converter (this converter is also referred to as a second frequency/voltage converter) 163 converts the frequency of the frequency-divided signal CLKD into a voltage signal V2 (this signal is also referred to as a second signal). The frequency/voltage converter 163 is configured in a way similar to the way the frequency/voltage converter 162 shown in Fig. 5 is configured.

The comparator 164 compares the voltage signal V2 with a predetermined voltage Vth and outputs a signal Vc, which is the result of the comparison, to the voltage holding circuit 166 and the switch 167 as a switch signal.

The voltage signal V1 is input to one input of the differential amplifier 165, and the voltage signal V2 is input to the other input of the differential amplifier 165. For example, in this embodiment, the voltage signal V2 is input to the inverting input of the differential amplifier 165, and the voltage signal V1 is input to the non-inverting input of the differential amplifier 165. Then the differential amplifier 165 outputs an output voltage Vd indicating the differential voltage between the voltage signal V1 and the voltage signal V2.

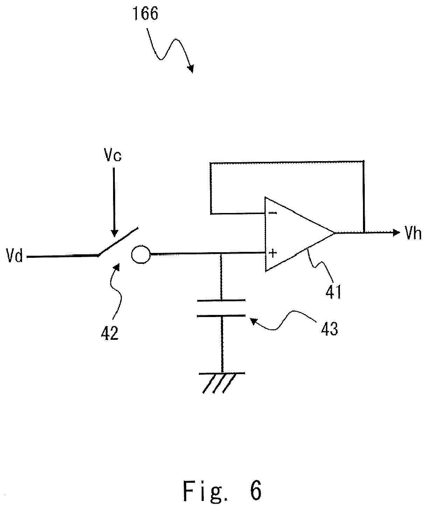

The voltage holding circuit 166 holds the output voltage Vd of the differential amplifier 165 in accordance with the signal Vc. Fig. 6 shows one example of the configuration of the voltage holding circuit. A switch 42 is controlled to be turned on or off by the signal Vc. When the switch 42 is in the ON state, the output voltage Vd is applied to a capacitor 43. When the switch 42 is in the OFF state, the voltage value of the output voltage Vd is held in the capacitor 43. The voltage value that has been held is output by a voltage follower circuit that uses the operational amplifier 41 as a voltage Vh.

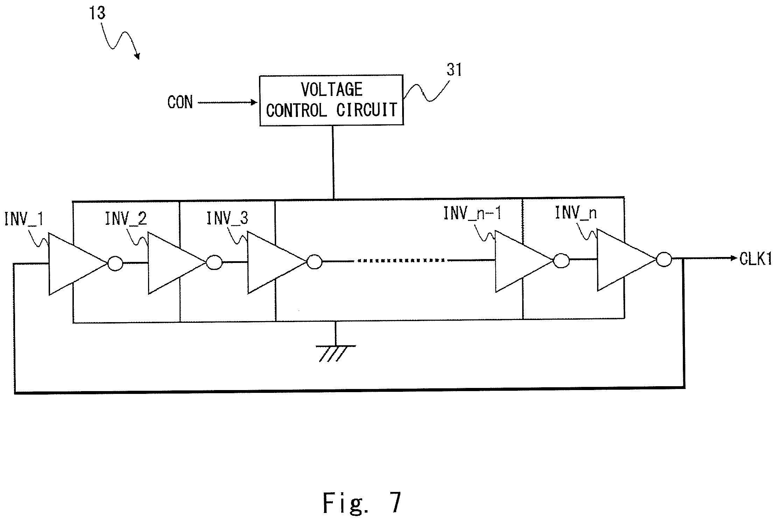

The switch 167 connects a control terminal of the oscillator 13 and one of the output terminal of the differential amplifier 165 and the output terminal of the voltage holding circuit 166 in accordance with the signal Vc. Fig. 7 shows one example of the configuration of the oscillator 13. The oscillator 13 is composed of a ring oscillator in which n (n is a positive odd number) inverting circuits INV_1-INV_n are connected in a ring shape. The power supply voltage is supplied from a voltage controller to each of the inverting circuits. The voltage value supplied from the voltage control circuit 31 is controlled by the control signal CON, which causes the delay amount of each inverting circuit to be changed, whereby the frequency of the clock signal CLK1 output from the oscillator 13 is controlled.

As described above, the clock signal generator 22 of the microcomputer 2 is not operated in synchronization with the sensor 1. Therefore, the frequency of the clock signal CLK1 fluctuates regardless of the clock signal CLK2. In addition, as described above, the frequency of the clock signal CLK1 tends to fluctuate relatively easily. Therefore, in order to guarantee the accuracy of the time series processing of the data of the microcomputer 2, the frequency of the clock signal CLK1 needs to be maintained at a predetermined value based on the clock signal CLK2. When the oscillation frequency of the clock signal CLK1 received from the microcomputer 2 cannot be known by the sensor 1 in advance, the frequency information of the clock used for the communication may be transmitted from the microcomputer 2 to the sensor 1. The transmission of the frequency information may be performed at a suitable timing.

In the following description, the operation of correcting the frequency of the clock signal CLK1 will be explained.

[1. When the clock signal CLK2 is input to the sensor 1]

When the clock signal CLK2 is input to the sensor 1, the frequency/voltage converter 163 outputs the voltage signal V2 indicating the frequency of the frequency-divided signal CLKD.

The predetermined voltage Vth input to the comparator 164 is set to such a value that the voltage signal V2 when the clock signal CLK2 is input becomes larger than the voltage Vth. Therefore, in this case, the comparator 164 outputs, for example, HIGH as the signal Vc.

When the signal Vc is HIGH, the switch 167 connects the control terminal of the oscillator 13 and the output terminal of the differential amplifier 165. Further, in the differential amplifier 165, the voltage signal V1 indicating the frequency of the clock signal CLK1 is compared with the voltage signal V2 indicating the frequency of the frequency-divided signal CLKD, and the output voltage Vd, which is the differential voltage between them, is output as the control signal CON.

The oscillator 13 increases or decreases the frequency of the clock signal CLK1 to be output in accordance with the value of the control signal CON, whereby the frequency of the clock signal CLK1 can be made coincide with the frequency of the frequency-divided signal CLKD.

[2. When the input of the clock signal CLK2 to the sensor 1 is stopped]

When the input of the clock signal CLK2 to the sensor 1 is stopped, the voltage signal V2 indicating the frequency of the frequency-divided signal CLKD becomes, for example, "0".

Therefore, the voltage signal V2 becomes smaller than the voltage Vth. Accordingly, the comparator 164 outputs, for example, LOW, as the signal Vc.

When the signal Vc is LOW, the voltage holding circuit 166 holds the control signal CON output from the differential amplifier 165 (that is, the output voltage Vd).

The switch 167 connects the control terminal of the oscillator 13 and the output terminal of the voltage holding circuit 166. Therefore, the control signal CON having the constant voltage Vh held by the voltage holding circuit 166 is input to the oscillator 13. Accordingly, the operation of correcting the frequency of the clock signal CLK1 in the oscillator 13 is interrupted.

[3. When the clock signal CLK2 is again input to the sensor 1]

In this case, as described above, the operation of correcting the frequency of the clock signal CLK1 in the oscillator 13 is restarted.

Next, Examples of the operation of correcting the clock signal CLK1 will be explained.

[Example 1: When the sensor 1 has unexpectedly entered the sleep mode]

The sensor 1 may enter the sleep mode in order to, for example, suppress power consumption. In this case, in order for the microcomputer 2 to read out data from the sensor 1, the sensor 1 is started and the clock signal CLK1 is corrected.

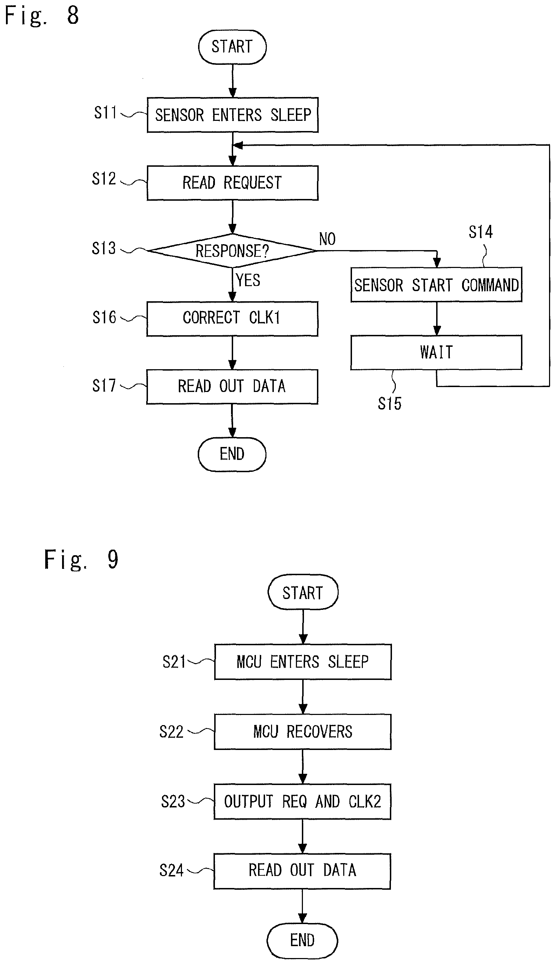

Fig. 8 is a diagram showing a recovery operation when the sensor 1 has unexpectedly entered the sleep mode.

Step S11

The sensor 1 unexpectedly enters the sleep mode.

Step S12

The microcomputer 2 sends the read request REQ to read the data stored in the memory 15 to the sensor 1.

Step S13

The microcomputer 2 determines whether there has been a response to the read request from the sensor 1.

Step S14

When there has been no response to the read command from the sensor 1, the microcomputer 2 sends a start command to the sensor 1.

Step S15

After a predetermined period of time has passed since the microprocessor 2 has sent the start command, the process goes back to Step S12.

Step S16

When there has been a response to the read command from the sensor 1, the microcomputer 2 sends the clock signal CLK2 to the sensor 1. The sensor 1 refers to the clock signal CLK2 and performs the operation of correcting the clock signal CLK1. Accordingly, the frequency of the clock signal CLK1 is corrected to a desired value.

Step S17

The microcomputer 2 reads out predetermined sampling data SD from the sensor 1.

[Example 2: When the microcomputer 2 has unexpectedly entered the sleep mode]

The microcomputer 2 may enter the sleep mode in order to, for example, suppress power consumption. In this case, when the microcomputer 2 recovers from the sleep mode, the clock signal CLK1 needs to be corrected.
Fig. 9 is a diagram showing a recovery operation when the microcomputer 2 has unexpectedly entered the sleep mode.

Step S21

The microcomputer (MCU) 2 unexpectedly enters the sleep mode.

Step S22

The microcomputer (MCU) 2 recovers from the sleep mode.

Step S23

The microcomputer 2 sends the read request REQ to the sensor 1 and outputs the clock signal CLK2. The sensor 1 refers to the clock signal CLK2 and performs the operation of correcting the clock signal CLK1. Accordingly, the frequency of the clock signal CLK1 is corrected to a desired value.

Step S24

The microcomputer 2 reads out the predetermined sampling data SD from the sensor 1.
As discussed above, according to this configuration, even when the frequency of the clock signal CLK1 output from the oscillator 13 of the sensor 1 is fluctuated, the frequency of the clock signal CLK1 can be corrected to an appropriate value based on the clock signal CLK2 supplied from the microcomputer 2.
Further, when the microcomputer 2 reads out data from the plurality of sensors having a configuration similar to that of the sensor 1, in order to synchronize the sampling of data of the respective sensors, the respective frequencies of the clock signals CLK1 of the respective sensors need to be the same. On the other hand, in this configuration, the microcomputer 2 outputs the clock signal CLK2 to each of the sensors, and each of the sensors is able to correct the respective clock signal CLK1. Therefore, it is possible to easily correct the clock signal CLK1 of each sensor.
Furthermore, the microcomputer 2 may set the sampling frequency and the frequency of the clock signal CLK1 by outputting, for example, necessary signals to the sensor 1. For example, the microcomputer 2 sets the sampling frequency and the frequency of the clock signal CLK1 in the sensor 1, whereby the sensor 1 is able to set the frequency division ratio of the clock signal CLK2. It is needless to say that the microcomputer 2 can set the frequency division ratio itself of the clock signal CLK2 in the sensor 1.

Second Embodiment

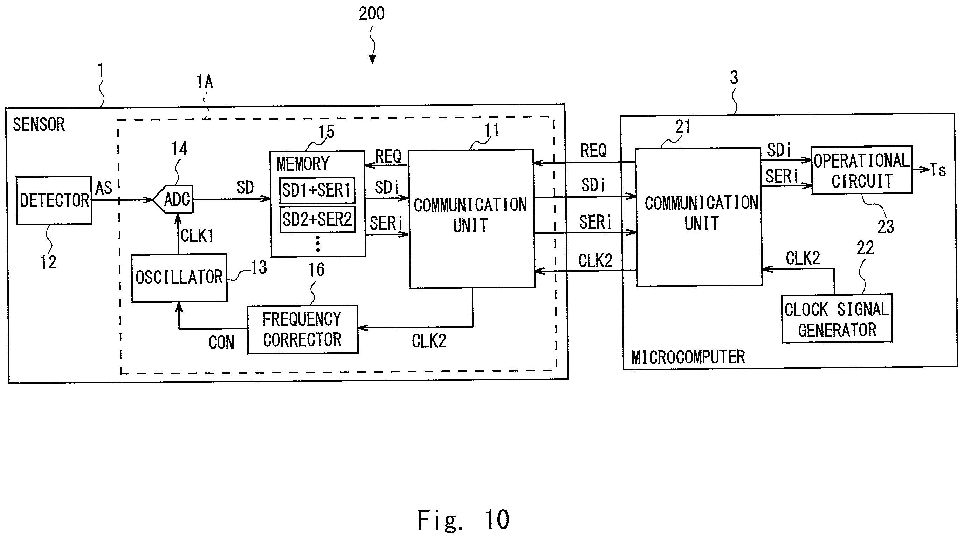

A detection system 100 according to a second embodiment will be explained. Fig. 10 is a diagram schematically showing a configuration of a detection system 200 according to the second embodiment. The detection system 200 has a configuration in which the microcomputer 2 of the detection system 100 according to the first embodiment is replaced by a microcomputer 3. The detection system 200 is configured as a system for calculating the sampling time of the data based on the information for calculating the time when the data has been sampled, the information having been received by the microcomputer 3 from the sensor 1. Further, the operation timing of the detection system 200 is similar to that shown in Fig. 3.
The microcomputer 3 includes, besides the components included in the microcomputer 2, an operation unit (operational circuit) 23. The operational circuit 23 can be composed of a logic circuit. Further, the operational circuit 23 can be achieved by a program executed by a CPU (not shown).

In this embodiment, the memory 15 stores the sampling data SD output from the A/D converter 14, and outputs, in accordance with the request REQ from the microcomputer 3, the sampling data SD which is to be output and serial data SER, which is information indicating the timing when the data has been sampled after the reset of the sampling data SD, via the communication unit 11.

In Fig. 10, as an example, the first sampling data after the reset is represented by SD1, the first serial data after the reset is represented by SER1, the second sampling data after the reset is represented by SD2, the second serial data after the reset is represented by SER2, ···, the i-th sampling data after the reset is represented by SDi, and the i-th serial data after the reset is represented by SERi.

Next, an operation for calculating the sampling time of the microcomputer 3 will be explained. The microcomputer 3 may reset the serial data SER of the memory 15 of the sensor 1 at the timing when the detection system 200 is started or at a desired timing.

Specifically, the microcomputer 3 outputs a reset signal RS. The reset signal RS is input to the memory 15, and the serial data SER is reset to be "0". After that, the serial data SER is incremented by the memory 15 every time it receives the sampling data, and adds the serial data SER to the sampling data SD.

When the microcomputer 3 receives the sampling data SD and the serial data SER, the operational circuit 23 refers to the serial data SER and calculates the time when the corresponding sampling data SD has been sampled. Hereinafter, the calculation method will be explained.

As shown in the following Expression [1], the microcomputer 3 adds a value obtained by multiplying a sampling period Ps by the value N of the serial data SER indicating the number of times of sampling to the reference time Tref, thereby calculating the sampling time Ts. The reference time Tref is a time when the microcomputer 3 has output the reset signal RS to the sensor 1. The operational circuit 23 holds, for example, the time when the reset signal RS has been output, as the reference time Tref, whereby it is able to refer to the reference time Tref as necessary.

$$Ts=Tref+N \cdot Ps \quad [1]$$

Further, it is also possible to calculate the sampling time more accurately. For example, there is a delay time required for transmission of signals and processing of signals between the timing when the data is actually sampled and the timing when the microcomputer 3 calculates the sampling time. The delay time by the A/D conversion processing in the A/D converter 14 is represented by TD1. The delay time by the A/D conversion processing here means time required from a rising edge or a falling edge of the clock signal CLK1 to start of the A/D conversion. Further, the delay time required for the time synchronization is represented by TD2. The delay time TD2 required for the time synchronization is a time required from the time when the microcomputer 3 outputs the reset signal RS to the time when the reset of the serial data SER by the memory 15 is completed. In this case, the sampling time Ts can be calculated by the following Expression [2].

$$Ts=Tref+N \cdot Ps+TD1+TD2 \quad [2]$$

While the delay time TD1 and the delay time TD2 have been assumed here, it is needless to say that a delay time caused by another factor may be added as appropriate.

Further, the information regarding the sampling period and the delay time used for the calculations may be stored in, for example, a memory (not shown) provided in the microcomputer 3. Further, the sampling period may be set in the sensor 1 by supplying the signal indicating the sampling period from the memory of the microcomputer 3 to the sensor 1.

As described above, according to this configuration, besides correcting the frequency of the clock signal CLK1 of the sensor 1, the microcomputer 3 is able to calculate the sampling time of the sampling data SD based on the serial data SER. It is therefore possible to correctly obtain the time series of the sampling data SD.

Further, according to this configuration, the serial data SER is incremented every time the sampling is performed in the sensor 1. Therefore, when the sampling data that the microcomputer 3 receives from the sensor 1 is missing, this causes the value of the serial data SER to be missing as well. Therefore, the presence of the sampling data which has failed to be obtained can be easily detected. In this case, for example, the microcomputer 3 may request the sensor 1 to output the sampling data which has failed to be obtained again.

When the microcomputer 3 reads out the sampling data from the plurality of sensors having the configuration similar to that of the sensor 1, in order to associate the sampling data sampled at the same time in the respective sensors with one another in the microcomputer 3, the microcomputer 3 needs to refer to the sampling time of the sampling data of the respective sensors. On the other hand, according to this configuration, as described above, the sampling time of the sampling data of the respective sensors can be calculated, whereby it is possible to easily associate the sampling data sampled at the same time in the plurality of sensors with one another.

According to this configuration, in order to calculate the sampling time in the microcomputer 3, the serial data SER is output from the sensor 1 to the microcomputer 3. Therefore, for example, there is no need to provide a part that outputs the data indicating the time in the sensor 1 and the time data indicating the sampling time itself needs not be transmitted from the sensor 1 to the microcomputer 3. Therefore, this configuration is advantageous in that it is possible to reduce the size of the sensor and to improve the compression rate of the data to be output from the sensor to the microcomputer.

Third Embodiment

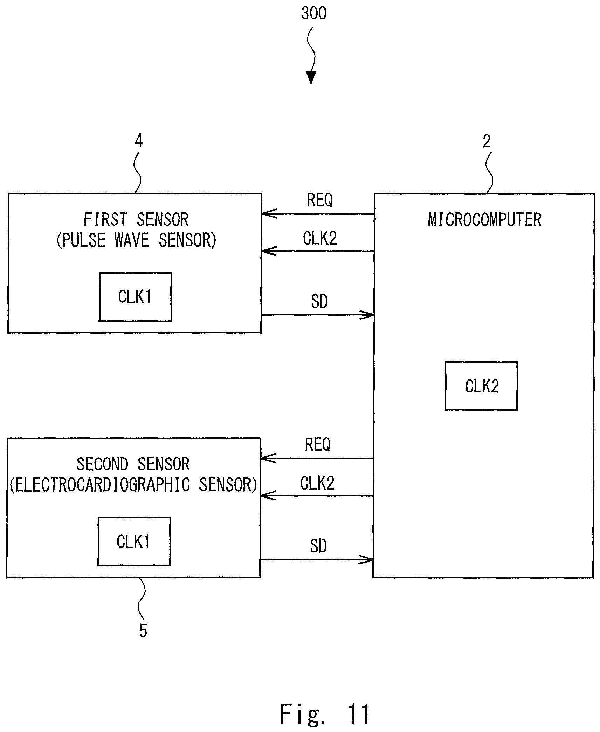

A detection system 300 according to a third embodiment will be explained. Fig. 11 is a diagram schematically showing a configuration of the detection system 300 according to the third embodiment. The detection system 300 includes a plurality of sensors. The detection system 300 includes a microcomputer 2, a first sensor 4, and a second sensor 5. In this example, the sensors 4 and 5 have a configuration similar to that of the sensor 1 of the detection system 100. The microcomputer 2 is similar to that of the detection system 100.

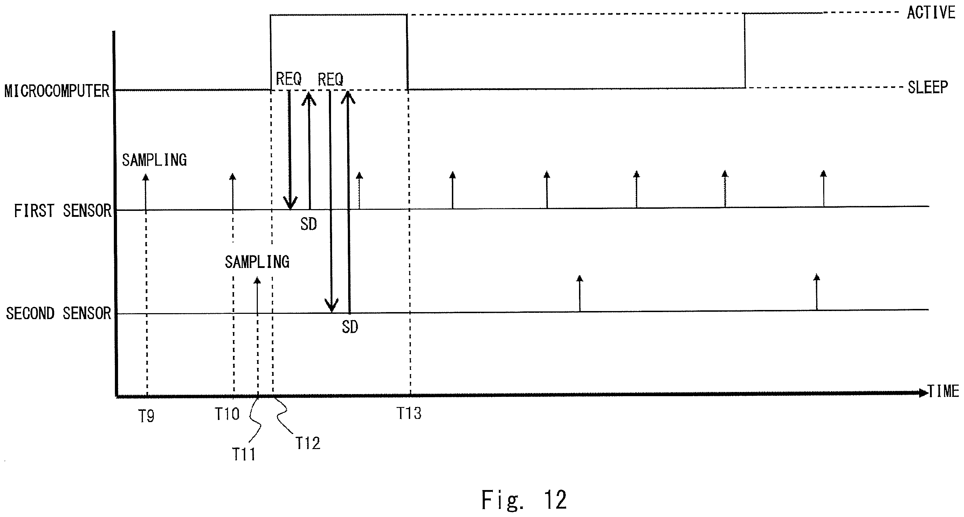

Fig. 12 shows one example of the operation timing. The first sensor 4 holds the sampling data SD at time T9 and time T10 in the memory. The second sensor 5 holds the sampling data at time T11 in the memory. Next, after the microcomputer 2 enters the active mode from the sleep mode at time T12, the clock signal CLK2 and the read request REQ are output to the first sensor 4. Upon receiving the read request REQ, the first sensor 4 outputs the sampling data SD at time T9 and T10 to the microcomputer 2. Further, the clock signal CLK2 and the read request REQ are output to the second sensor 5. Upon receiving the read request REQ, the second sensor 5 outputs the sampling data SD at time T11 to the microcomputer 2. Then the microcomputer 2 enters the sleep mode at time T13.

The detection system 300 is, for example, a system for acquiring and processing biological information. For example, the sensor 4 is a pulse wave sensor, detects biological pulse waves, and outputs the result of the detection as sampling data SD4. For example, the sensor 5 is an electrocardiographic sensor, detects an electrocardiogram of a living body, and outputs the result of the detection as sampling data SD5. The microcomputer 2 estimates the propagation speed of the pulse waves from the phase difference between the peak of the electrocardiogram and the peak of the pulse waves based on the sampling data SD4 and the sampling data SD5.

Accordingly, in order to secure the estimation accuracy of the propagation speed of the pulse wave, the accuracy of the time of the data sampled by the sensor becomes important. On the other hand, according to this configuration, the clock frequencies of the sensors 4 and 5 can be corrected in a way similar to those in the detection system 100, whereby it is possible to increase the processing accuracy of the sampling data.

In this embodiment, the microcomputer 2 may be replaced by the microcomputer 3 according to the second embodiment. In this case, the sampling time of the sampling data received from the plurality of sensors can be calculated, whereby it is possible to compare the plurality of pieces of time series data more reliably and more accurately.

Fourth Embodiment

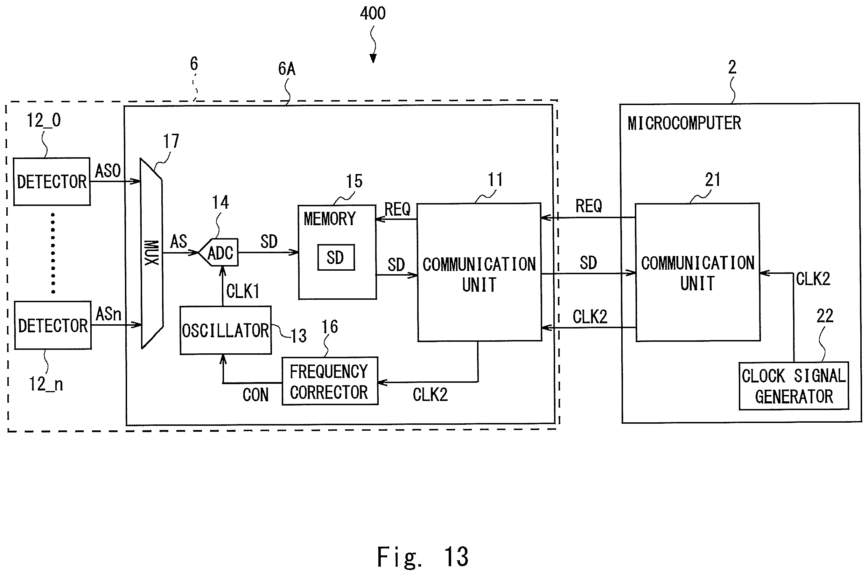

A detection system 400 according to a fourth embodiment will be explained. Fig. 13 is a diagram schematically showing a configuration of the detection system 400 according to the fourth embodiment. The detection system 400 includes a plurality of detectors 12_0-12_n (n is an integer equal to or larger than 1), a signal processor 6A, and a microcomputer 2. Since the microcomputer 2 is similar to that described in the first embodiment, descriptions thereof will be omitted.

The detectors 12_0-12_n may all have the same type or detectors of different types may be included. Analog signals ASO-ASn, which are signals output from the detectors 12_0-12_n, are input to the signal processor 6A.

The signal processor 6A includes a communication unit 11, an oscillator 13, an A/D converter 14, a memory 15, a frequency corrector 16, and a multiplexer 17. In this example, the communication unit 11, the oscillator 13, the A/D converter 14, the memory 15, the frequency corrector 16, and the multiplexer 17 are composed as an A/D conversion unit to perform A/D conversion on the analog signals ASO-ASn, which are signals output from the detectors 12_0-12_n. Since the communication unit 11, the oscillator 13, the A/D converter 14, the memory 15, and the frequency corrector 16 are similar to those of the first embodiment, descriptions thereof will be omitted.

The multiplexer 17 is configured to receive the analog signals ASO-ASn, which are the signals output from the detectors 12_0-12_n, and to output any one of the analog signals ASO-ASn to the A/D converter 14. In this case, the multiplexer 17 may receive, for example, the clock signal CLK1 and switch, based on the clock signal CLK1, the signal to be output among the analog signals ASO-ASn.

The multiplexer 17 may include a sample and hold function of the signal to be input to the multiplexer 17. In this case, the multiplexer 17 is able to output signals obtained by sampling the analog signals ASO-ASn while switching them as appropriate. Further, a sample and hold circuit composed of an analog switch and a capacitor may be provided, for example, between each of the detectors 12_0-12_n and the multiplexer 17. In this case, the analog switches may be concurrently turned on/off, so that a time difference will not be generated for each detector regarding the sampling timing of the analog signals AS0-ASn, which are signals output from the detectors 12_0-12_n. The timing when the analog signals AS0-ASn are sampled may be determined, for example, based on the clock signal CLK1.

Since the other operations of the detection system 400 are similar to those of the detection system 100 according to the first embodiment, descriptions thereof will be omitted.

In this example, the detectors 12_0-12_n and the signal processor 6A are physically separated from each other. However, it can be understood that the detectors 12_0-12_n and the signal processor 6A integrally compose a sensor 6 that corresponds to the sensor according to the aforementioned embodiments. In other words, the detector may be provided as an external component of the signal processor. By physically separating the detectors from the signal processor, the detector may be selected among the multiple detectors or one detector may be replaced by another one depending on the application, whereby the flexibility of the configuration of the detection system can be improved.

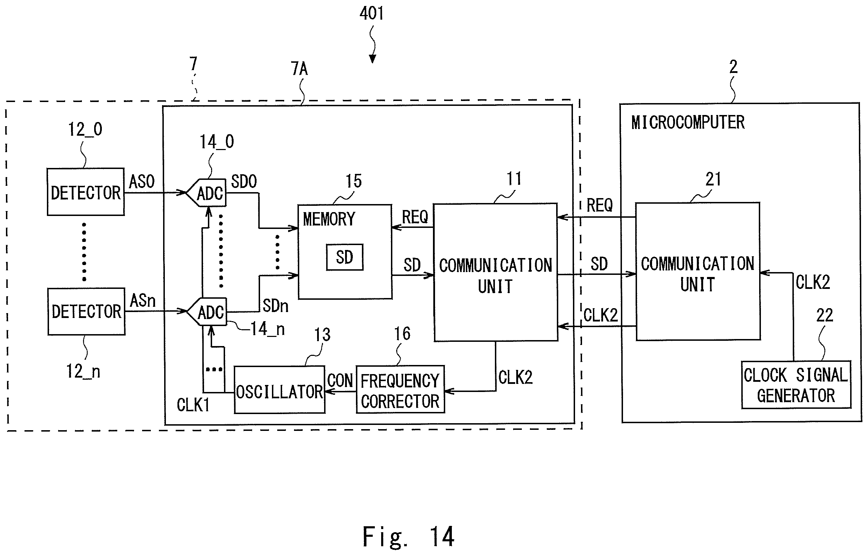

While the analog signals AS0-ASn from the detectors 12_0-12_n have been described as they are input to the multiplexer 17 in Fig. 13, a plurality of A/D converters may be provided in place of the multiplexer 17. Fig. 14 is a diagram schematically showing a configuration of a detection system 401, which is a modified example of the detection system 400 according to the fourth embodiment. As shown in Fig. 14, the detection system 401 includes a configuration in which the signal processor 6A of the detection system 400 is replaced by a signal processor 7A. The signal processor 7A includes a configuration in which the A/D converter 14 and the multiplexer 17 of the signal processor 6A are replaced by A/D converters 14_0-14_n.

The A/D converters 14_0-14_n respectively sample the analog signals AS0-ASn, convert the analog signals AS0-ASn into digital signals (sampling data SD0-SDn), and then output the digital signals (sampling data SD0-SDn) to the memory 15. In this case, only one of the A/D converters 14_0-14_n selectively outputs the sampling data based on the clock signal CLK1 supplied from the oscillator 13. Then the A/D converter that outputs the sampling data is switched in accordance with the clock signal CLK1, whereby the memory 15 is able to selectively receive one of the sampling data SD0-SDn output from the A/D converters 14_0-14_n and to sequentially hold the received sampling data.

Since the other operations of the detection system 401 are similar to those of the detection system 100 according to the first embodiment, descriptions thereof will be omitted.

In this case as well, the detectors 12_0-12_n are physically separated from the signal processor 7A. However, it can be understood that the detectors 12_0-12_n and the signal processor 7A integrally compose a sensor 7 that corresponds to the sensor according to the aforementioned embodiments. In other words, the detector may be provided as an external component of the signal processor. By physically separating the detectors from the signal processor, the detector may be selected among the multiple detectors or one detector may be replaced by another one, whereby the flexibility of the configuration of the detection system can be improved.

From the aforementioned discussion, according to this configuration, even when the plurality of detectors are provided, similar to the first embodiment, the sampling data can be output from the signal processor to the microcomputer in response to the request from the microcomputer.

While detection system 400 has been described above as a modified example of the detection system 100 according to the first embodiment, this is merely an example. It is needless to say that the plurality of detectors and the multiplexer may be provided also in the sensor of the detection system 200 according to the second embodiment. In the second embodiment, the example in which the operational circuit 23 of the microcomputer 3 calculates the sampling time Ts in consideration of the delay time TD1 and TD2 using Expression [2] has been explained. On the other hand, according to this configuration, the time required to switch the signals to be output from the multiplexer or the delay of the signal that occurs in the multiplexer itself and the like may further be added as the delay time to calculate the sampling time Ts.

Further, it is needless to say that the plurality of detectors and the plurality of A/D converters may be provided also in the sensor of the detection system 200 according to the second embodiment.

Further, it is needless to say that the sensor of the detection system 300 according to the third embodiment may be replaced by the plurality of detectors and the signal processor described in this embodiment as appropriate.

Fifth Embodiment

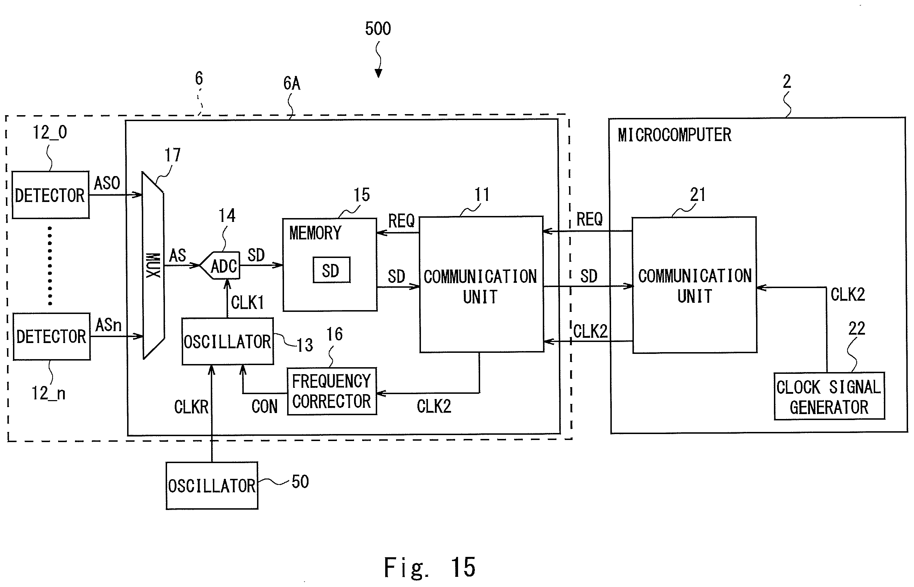

A detection system 500 according to a fifth embodiment will be explained. Fig. 15 is a diagram schematically showing a configuration of the detection system 500 according to the fifth embodiment. The detection system 500 is a modified example of the detection system 400 according to the fourth embodiment, and the reference clock CLKR referred to by the oscillator 13 is supplied to the oscillator 13 by the oscillator 50 provided outside of the signal processor 6A. Since the other configurations of the detection system 500 are similar to those of the detection system 400, descriptions thereof will be omitted.

According to this configuration, the oscillator 13 is able to output the clock signal CLK1 whose frequency has been adjusted in such a way that it is synchronized with the frequency of the clock signal CLK2 in the microcomputer by the control signal CON as appropriate based on the reference clock CLKR.

In this example, the detectors 12_0-12_n and the signal processor 6A are physically separated from each other. However, it can be understood that the detectors 12_0-12_n and the signal processor 6A integrally compose a sensor 6 that corresponds to the sensor according to the aforementioned embodiments, similar to the detection system 400. In other words, the detector may be provided as an external component of the signal processor. By physically separating the detectors from the signal processor, the detector may be selected among the multiple detectors or one detector may be replaced by another one depending on the application, whereby the flexibility of the configuration of the detection system can be improved.

While the detection system 500 has been described as a modified example of the detection system 400 according to the fourth embodiment, this is merely an example. That is, the oscillator 50 may be provided also in a detection system other than the detection system 400 according to the fourth embodiment.

Sixth Embodiment

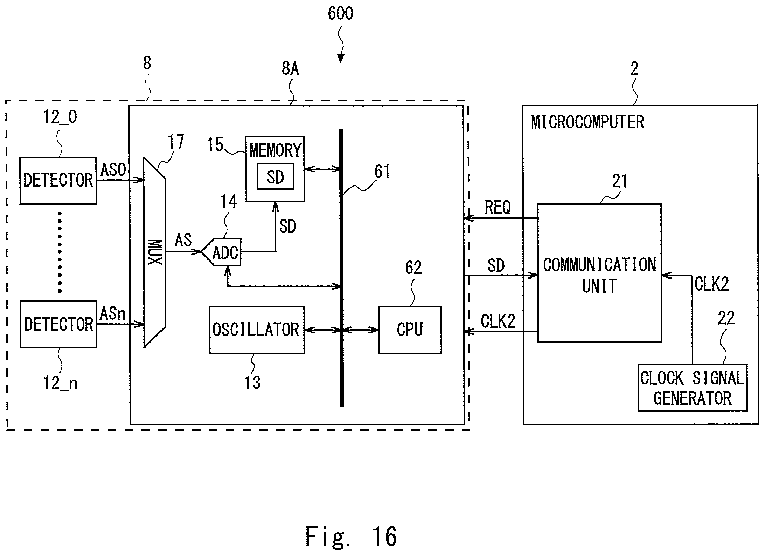

A detection system 600 according to a sixth embodiment will be explained. Fig. 16 is a diagram schematically showing a configuration of the detection system 600 according to the sixth embodiment. The detection system 600 includes a configuration in which the signal processor 6A of the detection system 400 according to the fourth embodiment is replaced by a Micro Control Unit (MCU) 8A, which is one aspect of the signal processor. Since the other configurations of the detection system 600 are similar to those of the detection system 400, descriptions thereof will be omitted.

The detection system 600 achieves the frequency correction function by the frequency corrector 16 of the detection system 400 by calculations performed by the CPU. Therefore, as shown in Fig. 16, in the MCU 8A of the detection system 600, the communication unit 11 and the frequency corrector 16 of the signal processor 6A of the detection system 400 are removed, and a bus 61 and a CPU 62 are instead provided. Since the oscillator 13, the A/D converter 14, the memory 15, and the multiplexer 17 are similar to those of the detection system 400, descriptions thereof will be omitted.

The bus 61 is configured in such a way that the address information and the data can be exchanged among the oscillator 13, the A/D converter 14, the memory 15, and the CPU 62.

The CPU 62 is configured to be able to output the control signal CON to control the oscillator 13 in such a manner that the frequency of the clock signal CLK1 is synchronized with the frequency of the clock signal CLK2 to the oscillator 13 via the bus 61 based on the clock signal CLK1 output from the oscillator 13 and the clock signal CLK2 output from the microcomputer 2.

In this example, the CPU 62 is able to receive the clock signal CLK1 output from the oscillator 13 and the clock signal CLK2 output from the microcomputer 2. Then the CPU 62 compares the clock signal CLK1 with the clock signal CLK2, detects the deviation between the frequencies of these clock signals, and outputs the control signal CON based on the result of the detection. The oscillator 13 adjusts the frequency of the clock signal CLK1 as appropriate in accordance with the control signal CON that has been received.

In this example, the detectors 12_0-12_n and the signal processor 8A are physically separated from each other. However, it can be understood that the detectors 12_0-12_n and the signal processor 8A integrally compose a sensor 8 that corresponds to the sensor according to the aforementioned embodiments. In other words, the detector may be provided as an external component of the signal processor. By physically separating the detectors from the signal processor, the detector may be selected among the multiple detectors or one detector may be replaced by another one depending on the application, whereby the flexibility of the configuration of the detection system can be improved.

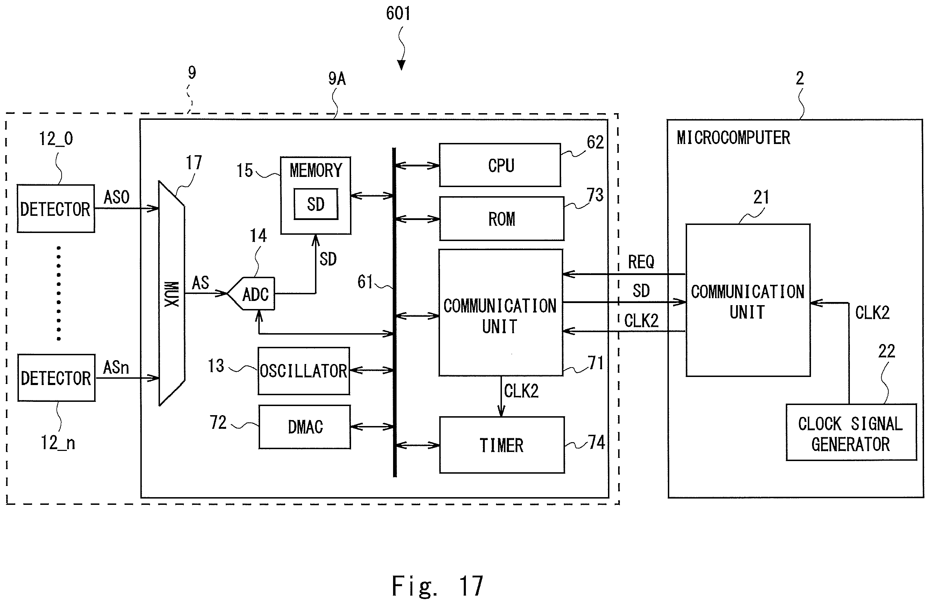

Next, a modified example of the detection system 600 will be explained. Fig. 17 is a diagram schematically showing a configuration of a detection system 601, which is a modified example of the detection system 600 according to the sixth embodiment. The detection system 601 includes a configuration in which the MCU 8A of the detection system 600 is replaced by an MCU 9A, which is one form of the signal processor.

The MCU 9A further includes, besides the components provided in the MCU 8A, a communication unit 71, a Direct Memory Access Controller (DMAC) 72, a Read Only Memory (ROM) 73, and a timer 74.

The communication unit 71 is connected to the bus 61, and includes a function similar to that of the communication unit 11 described above.

The DMAC 72 may perform the data transfer performed via the CPU, and is able to execute, for example, the data transfer from the memory 15 to the communication unit 71 in place of the CPU 62. It is therefore possible to reduce the load of the data transfer performed by the CPU 62. The data transfer carried out by the DMAC is not limited to this example.

The ROM 73 stores, for example, a program that defines the processing in the CPU 62 and parameters used for the processing, and the CPU 62 is able to read out the program or the parameter from the ROM 62 as necessary.

The timer 74 receives the clock signal CLK1 output from the oscillator 13 via the bus 61. Further, the timer 74 receives the clock signal CLK2 output from the microcomputer 2 via the communication unit 71 and the bus 61. The timer 74 is able to detect the pulse widths and the frequencies of the clock signals CLK1 and CLK2 by a timer function. Accordingly, the timer 74 detects the deviation of the frequency of the clock signal CLK1 with respect to the frequency of the clock signal CLK2. The CPU 62 receives data DET indicating the deviation of the frequency of the clock signal CLK1 detected by the timer 74 via the bus 61, and outputs the control signal CON to the oscillator 13 in accordance with the data DET, whereby it is possible to synchronize the frequency of the clock signal CLK1 with respect to the frequency of the clock signal CLK2.

Since the other configurations and the operations of the detection system 601 are similar to those of the detection system 400, descriptions thereof will be omitted.

In this example, the detectors 12_0-12_n and the signal processor 9A are physically separated from each other. However, it can be understood that the detectors 12_0-12_n and the signal processor 9A integrally compose a sensor 9 that corresponds to the sensor according to the aforementioned embodiments. In other words, the detector may be provided as an external component of the signal processor. By physically separating the detectors from the signal processor, the detector may be selected among the multiple detectors or one detector may be replaced by another one depending on the application, whereby the flexibility of the configuration of the detection system can be improved.

According to this configuration, in place of the frequency corrector 16 composed of the electric circuit, calculation processing by a CPU or a microcomputer is applied, whereby it is possible to synchronize the frequency of the clock signal CLK1 with respect to the frequency of the clock signal CLK2, similar to the first to fifth embodiments.

It is needless to say that the time required to switch the signals to be output from the multiplexer or the signal delay that occurs in the multiplexer itself and the like may further be added as a delay time to calculate the sampling time Ts in this configuration as well, similar to the fourth embodiment. Further, similar to the fifth embodiment, the reference clock CLKR referred to by the oscillator 13 may be supplied to the oscillator 13 by the oscillator provided outside of the signal processor.

Further, it is needless to say that the A/D converters 14_0-14_n that correspond to the detectors 12_0-12_n may be provided in the MCUs 8A and 9A as well in place of the A/D converter 14 and the multiplexer 17, similar to the detection system 401.

Other embodiments

Note that the present disclosure is not limited to the embodiments stated above and may be changed as appropriate without departing from the spirit of the present disclosure. While the sensor has been described that it includes the detectors and the signal processor in the first to third embodiments, the configuration of the detection system is not limited to this example. Similar to the fourth to sixth embodiments, it is needless to say that the detectors and the signal processor can be physically separated from each other. In other words, the detector may be provided as an external component of the signal processor. By physically separating the detectors from the signal processor, the detector may be selected among the multiple detectors or one detector may be replaced by another one depending on the application, whereby the flexibility of the configuration of the detection system can be improved.

While information such as data, clock signals, and requests are transmitted and received between the sensor or the signal processor and the microcomputer in the aforementioned embodiments, this transmission and reception of the information may be performed either by wired communication or wireless communication.

While the serial data SER has been described in the second embodiment, the serial data SER may not be added to all the pieces of the sampling data SD that the sensor outputs. The serial data SER may be added to the sampling data SD each time the sampling data SD is output a predetermined number of times. For example, the serial data SER, which is incremented by "10" as compared to the previous data output, may be added every time the sampling data SD is output ten times. Then the microcomputer 3 adds the integral multiple of the sampling period to the calculated sampling time, thereby being able to calculate the sampling time of the output data to which the serial data SER is not added.

In the aforementioned description, in the second embodiment, not only the correction of the frequency of the clock signal CLK1 according to the first embodiment but also the calculation of the sampling time of the data based on the serial data SER have been described. However, the configuration in which the sampling time is calculated based on the serial data SER described in the second embodiment does not presuppose the presence of the configuration of correcting the frequency of the clock signal CLK1 according to the first embodiment. That is, the absence of the configuration of correcting the frequency of the clock signal CLK1 according to the first embodiment does not inhibit the achievement of the detection system that includes the configuration of calculating the sampling time according to the second embodiment.

While the disclosure made by the present inventors has been described in detail based on the embodiments, it is needless to say that the present disclosure is not limited to the embodiments already stated above and may be changed in various ways without departing from the spirit of the present disclosure.

While the configuration of the frequency corrector has been described with reference to Fig. 4 in the first embodiment, this is merely an example. Another configuration may be applied as appropriate as long as a similar control signal can be output to the oscillator.

The first and second embodiments can be combined as desirable by one of ordinary skill in the art.

While the embodiments have been described above, the aforementioned detection system, the sensor, the microcomputer, and the method of correcting the detection system may be described as follows.

(Supplementary Note 1) A detection system comprising: a sensor configured to be able to output sampling data, which is a digital signal generated by performing analog/digital conversion on an analog signal indicating a detection result, the analog signal being sampled based on a first clock signal; and a microcomputer configured to generate a second clock signal and output the second clock signal to the sensor, and read out the sampling data from the sensor, wherein the sensor corrects a frequency of the first clock signal based on the second clock signal.

(Supplementary Note 2) The detection system according to Supplementary Note 1, wherein the sensor comprises: a first input/output unit (a first input/output interface) configured to perform data communication with the microcomputer; a detector configured to output the result of the detection as the analog signal; an oscillator configured to output the first clock signal whose frequency has been corrected based on a supplied control signal; a frequency corrector configured to output the control signal based on the second clock signal received from the microcomputer via the first input/output interface; an analog/digital converter configured to sample the analog signal based on the first clock signal, perform analog/digital conversion on the sampled analog signal, and output the sampling data; and a memory configured to store the sampling data, and the microcomputer comprises: a clock signal generator configured to generate the second clock signal; and a second input/output unit (a second input/output interface) configured to perform data communication with the sensor.

(Supplementary Note 3) The detection system according to Supplementary Note 2, wherein the microcomputer outputs the second clock signal when it reads out the sampling data from the memory.

(Supplementary Note 4) The detection system according to Supplementary Note 2, wherein the frequency corrector comprises: a frequency divider configured to divide a frequency of the second clock signal; a first frequency/voltage converter configured to output a first signal indicating a frequency of the first clock signal; a second frequency/voltage converter configured to output a second signal indicating the frequency of the signal divided by the frequency divider; a differential amplifier configured to output a signal indicating a differential voltage between the first signal and the second signal; a comparator configured to compare the second signal with a signal having a predetermined value and output a switch signal indicating the result of the comparison; a voltage holding circuit configured to hold the voltage of the signal output from the differential amplifier in accordance with the switch signal; a switch configured to connect the oscillator and one of an output of the differential amplifier and an output of the voltage holding circuit in accordance with the switch signal, the oscillator is connected to the output of the differential amplifier, which causes the voltage of the signal output from the differential amplifier to be input to the oscillator as the control signal, and the oscillator is connected to the output of the voltage holding circuit, which causes the voltage held by the voltage holding circuit to be input to the oscillator as the control signal.

(Supplementary Note 5) The detection system according to Supplementary Note 4, wherein the switch connects the oscillator and the output of the differential amplifier when the second clock signal is input from the microcomputer, and the switch connects the oscillator and the output of the voltage holding circuit when the second clock signal is not input from the microcomputer.

(Supplementary Note 6) The detection system according to Supplementary Note 2, wherein the microcomputer is able to output a reset signal to the sensor, the memory outputs serial data indicating the number of times of sampling of the data after the reset signal has been received to the microcomputer along with corresponding sampling data in accordance with a read request from the microcomputer and the microcomputer further comprises an operational circuit configured to calculate a sampling time of the received sampling data based on a reference time when the reset signal has been output, a sampling period in the sensor, and the received serial data.

(Supplementary Note 7) The detection system according to Supplementary Note 6, wherein the operational circuit adds a value obtained by multiplying the value of the received serial data by the sampling period to the reference time to calculate the sampling time.

(Supplementary Note 8) The detection system according to Supplementary Note 7, wherein the operational circuit further adds a delay time from when the operational circuit outputs the reset signal to when the serial data is reset to calculate the sampling time.

(Supplementary Note 9) The detection system according to Supplementary Note 7, wherein the operational circuit further adds a delay time that is required to convert the analog signal into the sampling data based on the first clock signal to calculate the sampling time.

(Supplementary Note 10) The detection system according to Supplementary Note 6, wherein the serial data is output to the microcomputer along with corresponding sampling data every time reading is requested from the microcomputer.

(Supplementary Note 11) The detection system according to Supplementary Note 6, wherein the serial data is output to the microcomputer along with corresponding sampling data every time reading is requested from the microcomputer a predetermined number of times.

(Supplementary Note 12) The detection system according to Supplementary Note 6, comprising a plurality of sensors, wherein the detection system associates sampling data sampled by the plurality of sensors at the same time with one another based on the calculated sampling time.

(Supplementary Note 13) A sensor configured to be able to output sampling data, which is a digital signal generated by performing analog/digital conversion on an analog signal indicating a detection result, the analog signal being sampled based on a first clock signal, wherein the sensor corrects a frequency of the first clock signal based on a second clock signal generated by a microcomputer, and the sampling data is read out by the microcomputer.

(Supplementary Note 14) The sensor according to Supplementary Note 13, comprising: a first input/output interface configured to perform data communication with the microcomputer; a detector configured to output the result of the detection as the analog signal; an oscillator configured to output the first clock signal whose frequency has been corrected based on a supplied control signal; a frequency corrector configured to output the control signal based on the second clock signal received from the microcomputer via the first input/output interface; an analog/digital converter configured to sample the analog signal based on the first clock signal, perform analog/digital conversion on the sampled analog signal, and output the sampling data; and a memory configured to store the sampling data.

(Supplementary Note 15) The sensor according to Supplementary Note 14, wherein the microcomputer comprises a clock signal generator configured to generate the second clock signal and a second input/output interface configured to perform data communication with the sensor.

(Supplementary Note 16) The sensor according to Supplementary Note 14, wherein the microcomputer outputs the second clock signal when it reads out the sampling data from the memory.

(Supplementary Note 17) The sensor according to Supplementary Note 14, wherein the frequency corrector comprises: a frequency divider configured to divide a frequency of the second clock signal; a first frequency/voltage converter configured to output a first signal indicating a frequency of the first clock signal; a second frequency/voltage converter configured to output a second signal indicating the frequency of the signal divided by the frequency divider; a differential amplifier configured to output a signal indicating a differential voltage between the first signal and the second signal; a comparator configured to compare the second signal with a signal having a predetermined value and output a switch signal indicating the result of the comparison; a voltage holding circuit configured to hold the voltage of the signal output from the differential amplifier in accordance with the switch signal; and a switch configured to connect the oscillator and one of an output of the differential amplifier and an output of the voltage holding circuit in accordance with the switch signal, wherein the oscillator is connected to the output of the differential amplifier, which causes the voltage of the signal output from the differential amplifier to be input to the oscillator as the control signal, and the oscillator is connected to the output of the voltage holding circuit, which causes the voltage held by the voltage holding circuit to be input to the oscillator as the control signal.

(Supplementary Note 18) The sensor according to Supplementary Note 17, wherein the switch connects the oscillator and the output of the differential amplifier when the second clock signal is input from the microcomputer, and the switch connects the oscillator and the output of the voltage holding circuit when the second clock signal is not input from the microcomputer.

(Supplementary Note 19) The sensor according to Supplementary Note 14, wherein the microcomputer is able to output a reset signal to the sensor, the memory outputs serial data indicating the number of times of sampling of the data after the reset signal has been received to the microcomputer along with corresponding sampling data in accordance with a read request from the microcomputer, and the microcomputer further comprises an operational circuit configured to calculate a sampling time of the received sampling data based on a reference time when the reset signal has been output, a sampling period in the sensor, and the received serial data.

(Supplementary Note 20) The sensor according to Supplementary Note 19, wherein the operational circuit adds a value obtained by multiplying the value of the received serial data by the sampling period to the reference time to calculate the sampling time.

(Supplementary Note 21) The sensor according to Supplementary Note 20, wherein the operational circuit further adds a delay time from when the operational circuit outputs the reset signal to when the serial data is reset to calculate the sampling time.

(Supplementary Note 22) The sensor according to Supplementary Note 20, wherein the operational circuit further adds a delay time that is required to convert the analog signal into the sampling data based on the first clock signal to calculate the sampling time.

(Supplementary Note 23) The sensor according to Supplementary Note 19, wherein the serial data is output to the microcomputer along with corresponding sampling data every time reading is requested from the microcomputer.

(Supplementary Note 24) The sensor according to Supplementary Note 19, wherein the serial data is output to the microcomputer along with corresponding sampling data every time reading is requested from the microcomputer a predetermined number of times.

(Supplementary Note 25) The sensor according to Supplementary Note 19, wherein the sensor associates sampling data sampled by the plurality of sensors at the same time with one another based on the calculated sampling time.

(Supplementary Note 26) A microcomputer configured to generate a second clock signal and output the second clock signal to a sensor configured to be able to output sampling data, which is a digital signal generated by performing analog/digital conversion on an analog signal indicating a detection result, the analog signal being sampled based on a first clock signal, the microcomputer being further configured to be able to read out the sampling data from the sensor, wherein a frequency of the first clock signal is corrected by the sensor based on the second clock signal.

(Supplementary Note 27) The microcomputer according to Supplementary Note 26, comprising: a clock signal generator configured to generate the second clock signal; and a second input/output interface configured to perform data communication with the sensor.

(Supplementary Note 28) The microcomputer according to Supplementary Note 26, wherein the sensor comprises: a first input/output interface configured to perform data communication with the microcomputer; a detector configured to output the result of the detection as the analog signal; an oscillator configured to output the first clock signal whose frequency has been corrected based on a supplied control signal; a frequency corrector configured to output the control signal based on the second clock signal received from the microcomputer via the first input/output interface; an analog/digital converter configured to sample the analog signal based on the first clock signal, perform analog/digital conversion on the sampled analog signal, and output the sampling data; and a memory configured to store the sampling data.

(Supplementary Note 29) The microcomputer according to Supplementary Note 28, wherein the microcomputer outputs the second clock signal when it reads out the sampling data from the memory.

(Supplementary Note 30) The microcomputer according to Supplementary Note 28, wherein the frequency corrector comprises: a frequency divider configured to divide a frequency of the second clock signal; a first frequency/voltage converter configured to output a first signal indicating a frequency of the first clock signal; a second frequency/voltage converter configured to output a second signal indicating the frequency of the signal divided by the frequency divider; a differential amplifier configured to output a signal indicating a differential voltage between the first signal and the second signal; a comparator configured to compare the second signal with a signal having a predetermined value and output a switch signal indicating the result of the comparison; a voltage holding circuit configured to hold the voltage of the signal output from the differential amplifier in accordance with the switch signal; and a switch configured to connect the oscillator and one of an output of the differential amplifier and an output of the voltage holding circuit in accordance with the switch signal, wherein the oscillator is connected to the output of the differential amplifier, which causes the voltage of the signal output from the differential amplifier to be input to the oscillator as the control signal, and the oscillator is connected to the output of the voltage holding circuit, which causes the voltage held by the voltage holding circuit to be input to the oscillator as the control signal.

(Supplementary Note 31) The microcomputer according to Supplementary Note 30, wherein the switch connects the oscillator and the output of the differential amplifier when the second clock signal is input from the microcomputer, and the switch connects the oscillator and the output of the voltage holding circuit when the second clock signal is not input from the microcomputer.

(Supplementary Note 32) The microcomputer according to Supplementary Note 28, wherein the microcomputer is able to output a reset signal to the sensor, the memory outputs serial data indicating the number of times of sampling of the data after the reset signal has been received to the microcomputer along with corresponding sampling data in accordance with a read request from the microcomputer, and the microcomputer further comprises an operational circuit configured to calculate a sampling time of the received sampling data based on a reference time when the reset signal has been output, a sampling period in the sensor, and the received serial data.

(Supplementary Note 33) The microcomputer according to Supplementary Note 32, wherein the operational circuit adds a value obtained by multiplying the value of the received serial data by the sampling period to the reference time to calculate the sampling time.

(Supplementary Note 34) The microcomputer according to Supplementary Note 33, wherein the operational circuit further adds a delay time from when the operational circuit outputs the reset signal to when the serial data is reset to calculate the sampling time.

(Supplementary Note 35) The microcomputer according to Supplementary Note 33, wherein the operational circuit further adds a delay time that is required to convert the analog signal into the sampling data based on the first clock signal to calculate the sampling time.

(Supplementary Note 36) The microcomputer according to Supplementary Note 32, wherein the serial data is output to the microcomputer along with corresponding sampling data every time reading is requested from the microcomputer.

(Supplementary Note 37) The microcomputer according to Supplementary Note 32, wherein the serial data is output to the microcomputer along with corresponding sampling data every time reading is requested from the microcomputer a predetermined number of times.

(Supplementary Note 38) The microcomputer according to Supplementary Note 32, comprising a plurality of sensors, wherein the microcomputer associates sampling data sampled by the plurality of sensors at the same time with one another based on the calculated sampling time.

(Supplementary Note 39) The microcomputer according to Supplementary Note 28, wherein the microcomputer outputs the second clock signal when it reads out the sampling data from the sensor.

(Supplementary Note 40) The microcomputer according to Supplementary Note 28, wherein the microcomputer is capable of outputting a reset signal to the sensor, the sensor outputs serial data indicating the number of times of sampling of the data after the reset signal has been received to the microcomputer along with corresponding sampling data in accordance with a read request from the microcomputer, and the microcomputer further comprises an operational circuit configured to calculate a sampling time of the received sampling data based on a reference time when the reset signal has been output, a sampling period in the sensor, and the received serial data.

(Supplementary Note 41) A method of correcting a detection system comprising: generating a second clock signal; outputting the second clock signal from a microcomputer that reads out sampling data from a sensor to the sensor configured to be able to output the sampling data, which is a digital signal generated by performing analog/digital conversion on an analog signal indicating a detection result, the analog signal being sampled based on a first clock signal, and causing the sensor to correct a frequency of the first clock signal based on the second clock signal.

(Supplementary Note 42) The method of correcting the detection system according to Supplementary Note 41, wherein the sensor comprises: a first input/output interface configured to perform data communication with the microcomputer; a detector configured to output the result of the detection as the analog signal; an oscillator configured to output the first clock signal whose frequency has been corrected based on a supplied control signal; a frequency corrector configured to output the control signal based on the second clock signal received from the microcomputer via the first input/output interface; an analog/digital converter configured to sample the analog signal based on the first clock signal, perform analog/digital conversion on the sampled analog signal, and output the sampling data; and a memory configured to store the sampling data, wherein the microcomputer comprises a clock signal generator configured to generate the second clock signal and a second input/output interface configured to perform data communication with the sensor.

(Supplementary Note 43) The method of correcting the detection system according to Supplementary Note 42, comprising causing the microcomputer to output the second clock signal when it reads out the sampling data from the memory.

(Supplementary Note 44) The method of correcting the detection system according to Supplementary Note 42, wherein the frequency corrector comprises: a frequency divider configured to divide a frequency of the second clock signal; a first frequency/voltage converter configured to output a first signal indicating a frequency of the first clock signal; a second frequency/voltage converter configured to output a second signal indicating the frequency of the signal divided by the frequency divider; a differential amplifier configured to output a signal indicating a differential voltage between the first signal and the second signal; a comparator configured to compare the second signal with a signal having a predetermined value and output a switch signal indicating the result of the comparison; a voltage holding circuit configured to hold the voltage of the signal output from the differential amplifier in accordance with the switch signal; and a switch configured to connect the oscillator and one of an output of the differential amplifier and an output of the voltage holding circuit in accordance with the switch signal, wherein the oscillator is connected to the output of the differential amplifier, which causes the voltage of the signal output from the differential amplifier to be input to the oscillator as the control signal, and the oscillator is connected to the output of the voltage holding circuit, which causes the voltage held by the voltage holding circuit to be input to the oscillator as the control signal.

(Supplementary Note 45) The method of correcting the detection system according to Supplementary Note 44, comprising causing the switch to connect the oscillator and the output of the differential amplifier when the second clock signal is input from the microcomputer, and causing the switch to connect the oscillator and the output of the voltage holding circuit when the second clock signal is not input from the microcomputer.

(Supplementary Note 46) The method of correcting the detection system according to Supplementary Note 44, wherein the microcomputer is able to output a reset signal to the sensor, the method causes the memory to output serial data indicating the number of times of sampling of the data after the reset signal has been received to the microcomputer along with corresponding sampling data in accordance with a read request from the microcomputer, and the method causes the microcomputer to calculate a sampling time of the received sampling data based on a reference time when the reset signal has been output, a sampling period in the sensor, and the received serial data.

(Supplementary Note 47) The method of correcting the detection system according to Supplementary Note 46, comprising adding a value obtained by multiplying the value of the received serial data by the sampling period to the reference time to calculate the sampling time.

(Supplementary Note 48) The method of correcting the detection system according to Supplementary Note 47, comprising further adding a delay time from when the reset signal is output to when the serial data is reset to calculate the sampling time.

(Supplementary Note 49) The method of correcting the detection system according to Supplementary Note 47, comprising further adding a delay time that is required to convert the analog signal into the sampling data based on the first clock signal to calculate the sampling time.

(Supplementary Note 50) The method of correcting the detection system according to Supplementary Note 46, wherein the serial data is output to the microcomputer along with corresponding sampling data every time reading is requested from the microcomputer.

(Supplementary Note 51) The method of correcting the detection system according to Supplementary Note 46, wherein the serial data is output to the microcomputer along with corresponding sampling data every time reading is requested from the microcomputer a predetermined number of times.

(Supplementary Note 52) The method of correcting the detection system according to Supplementary Note 46, wherein a plurality of sensors are provided and sampling data sampled by the plurality of sensors at the same time are associated with one another based on the calculated sampling time.

While the invention has been described in terms of several embodiments, those skilled in the art will recognize that the invention can be practiced with various modifications within the spirit and scope of the appended claims and the invention is not limited to the examples described above.

Further, the scope of the claims is not limited by the embodiments described above.

Furthermore, it is noted that, Applicant's intent is to encompass equivalents of all claim elements, even if amended later during prosecution.

## Claims

1.    A detection system comprising:

a sensor configured to be able to output sampling data, which is a digital signal generated by performing analog/digital conversion on an analog signal indicating a detection result, the analog signal being sampled based on a first clock signal; and
a microcomputer configured to generate a second clock signal and output the second clock signal to the sensor, and read out the sampling data from the sensor,
wherein the sensor corrects a frequency of the first clock signal based on the second clock signal.

2.    The detection system according to Claim 1, wherein the sensor comprises:

a first input/output interface configured to perform data communication with the microcomputer;
a detector configured to output the result of the detection as the analog signal;
an oscillator configured to output the first clock signal whose frequency has been corrected based on a supplied control signal;
a frequency corrector configured to output the control signal based on the second clock signal received from the microcomputer via the first input/output interface;
an analog/digital converter configured to sample the analog signal based on the first clock signal, perform analog/digital conversion on the sampled analog signal, and output the sampling data; and
a memory configured to store the sampling data, and

the microcomputer comprises:

a clock signal generator configured to generate the second clock signal; and
a second input/output interface configured to perform data communication with the sensor.

3.    The detection system according to Claim 2, wherein the microcomputer outputs the second clock signal when it reads out the sampling data from the memory.

4.    The detection system according to Claim 2, wherein the frequency corrector comprises:

a frequency divider configured to divide a frequency of the second clock signal;

a first frequency/voltage converter configured to output a first signal indicating a frequency of the first clock signal;

a second frequency/voltage converter configured to output a second signal indicating the frequency of the signal divided by the frequency divider;

a differential amplifier configured to output a signal indicating a differential voltage between the first signal and the second signal;

a comparator configured to compare the second signal with a signal having a predetermined value and output a switch signal indicating the result of the comparison;

a voltage holding circuit configured to hold the voltage of the signal output from the differential amplifier in accordance with the switch signal; and

a switch configured to connect the oscillator and one of an output of the differential amplifier and an output of the voltage holding circuit in accordance with the switch signal,

the oscillator is connected to the output of the differential amplifier, which causes the voltage of the signal output from the differential amplifier to be input to the oscillator as the control signal, and

the oscillator is connected to the output of the voltage holding circuit, which causes the voltage held by the voltage holding circuit to be input to the oscillator as the control signal.

5. The detection system according to Claim 4, wherein the switch connects the oscillator and the output of the differential amplifier when the second clock signal is input from the microcomputer, and

the switch connects the oscillator and the output of the voltage holding circuit when the second clock signal is not input from the microcomputer.

6. The detection system according to Claim 2, wherein the microcomputer is able to output a reset signal to the sensor,

the memory outputs serial data indicating the number of times of sampling of the data after the reset signal has been received to the microcomputer along with corresponding sampling data in accordance with a read request from the microcomputer, and

the microcomputer further comprises an operational circuit configured to calculate the sampling time of the received sampling data based on a reference time when the reset signal has been output, a sampling period in the sensor, and the received serial data.

7. The detection system according to Claim 6, wherein the operational circuit adds a value obtained by multiplying the value of the received serial data by the sampling period to the reference time to calculate the sampling time.

8. The detection system according to Claim 7, wherein the operational circuit further adds a delay time from when the operational circuit outputs the reset signal to when the serial data is reset to calculate the sampling time.

9. The detection system according to Claim 7, wherein the operational circuit further adds a delay time that is required to convert the analog signal into the sampling data based on the first clock signal to calculate the sampling time.

10. The detection system according to Claim 6, wherein the serial data is output to the microcomputer along with corresponding sampling data every time reading is requested from the microcomputer.

11. The detection system according to Claim 6, wherein the serial data is output to the microcomputer along with corresponding sampling data every time reading is requested from the microcomputer a predetermined number of times.

12. The detection system according to Claim 6, comprising a plurality of sensors, wherein the detection system associates sampling data sampled by the plurality of sensors at the same time with one another based on the calculated sampling time.

13. A sensor configured to be able to output sampling data, which is a digital signal generated by performing analog/digital conversion on an analog signal indicating a detection result, the analog signal being sampled based on a first clock signal, wherein the sensor corrects a frequency of the first clock signal based on a second clock signal generated by a microcomputer, and

the sampling data is read out by the microcomputer.

14. The sensor according to Claim 13, comprising:

a first input/output interface configured to perform data communication with the microcomputer;

a detector configured to output the result of the detection as the analog signal;

an oscillator configured to output the first clock signal whose frequency has been corrected based on a supplied control signal;

a frequency corrector configured to output the control signal based on the second clock signal received from the microcomputer via the first input/output interface;

an analog/digital converter configured to sample the analog signal based on the first clock signal, perform analog/digital conversion on the sampled analog signal, and output the sampling data; and

a memory configured to store the sampling data.

15. The sensor according to Claim 14, wherein the frequency corrector comprises:

a frequency divider configured to divide a frequency of the second clock signal;

a first frequency/voltage converter configured to output a first signal indicating a frequency of the first clock signal;

a second frequency/voltage converter configured to output a second signal indicating the frequency of the signal divided by the frequency divider;

a differential amplifier configured to output a signal indicating a differential voltage between the first signal and the second signal;

a comparator configured to compare the second signal with a signal having a predetermined value and output a switch signal indicating the result of the comparison;

a voltage holding circuit configured to hold the voltage of the signal output from the differential amplifier in accordance with the switch signal; and

a switch configured to connect the oscillator and one of an output of the differential amplifier and an output of the voltage holding circuit in accordance with the switch signal, wherein

the oscillator is connected to the output of the differential amplifier, which causes the voltage of the signal output from the differential amplifier to be input to the oscillator as the control signal, and

the oscillator is connected to the output of the voltage holding circuit, which causes the voltage held by the voltage holding circuit to be input to the oscillator as the control signal.

16. The sensor according to Claim 15, wherein the switch connects the oscillator and the output of the differential amplifier when the second clock signal is input from the microcomputer, and the switch connects the oscillator and the output of the voltage holding circuit when the second clock signal is not input from the microcomputer.

17. A microcomputer configured to generate a second clock signal and output the second clock signal to a sensor configured to be able to output sampling data, which is a digital signal generated by performing analog/digital conversion on an analog signal indicating a detection result, the analog signal being sampled based on a first clock signal, the microcomputer being further configured to be able to read out the sampling data from the sensor,

wherein a frequency of the first clock signal is corrected by the sensor based on the second clock signal.

18. The microcomputer according to Claim 17, comprising:

a clock signal generator configured to generate the second clock signal; and

a second input/output interface configured to perform data communication with the sensor.

19. The microcomputer according to Claim 18, wherein the microcomputer outputs the second clock signal when it reads out the sampling data from the sensor.

20. The microcomputer according to Claim 18, wherein the microcomputer is capable of outputting a reset signal to the sensor,

the sensor outputs serial data indicating the number of times of sampling of the data after the reset signal has been received to the microcomputer along with corresponding sampling data in accordance with a read request from the microcomputer, and

the microcomputer further comprises an operational circuit configured to calculate a sampling time of the received sampling data based on a reference time when the reset signal has been output, a sampling period in the sensor, and the received serial data.

100

1                                         2

| SENSOR | MICROCOMPUTER |
|---|---|

REQ

SD

CLK1            CLK2              CLK2

Fig. 1

Fig. 2

EP 3 416 291 A2

Fig. 3

Fig. 4

162

33

CLKIN →

TIMING
CONTROL CIRCUIT

CHR

DCHR

CONSTANT
CURRENT CIRCUIT

32

34

35

36

→ VOUT

Fig. 5

166

Vc

Vd

Vh

41

42

43

Fig. 6

EP 3 416 291 A2

Fig. 7

Fig. 8

```
              ( START )
                  │
                  ▼
S11 ──  SENSOR ENTERS SLEEP
                  │
                  ▼  ◄──────────────────────────┐
S12 ──     READ REQUEST                          │
                  │                              │
                  ▼                          S14 │
S13 ──  ◇ RESPONSE? ◇ ── NO ──────┐              │
                  │           ┌─────────────────┐│
                 YES          │ SENSOR START COMMAND │
                  │           └─────────────────┘│
                  ▼                   │          │
S16 ──   CORRECT CLK1                 ▼          │
                  │           ┌──────────────┐   │
                  ▼           │     WAIT     │───┘
S17 ──   READ OUT DATA        └──────────────┘
                  │             S15
                  ▼
               ( END )
```

Fig. 9

```
              ( START )
                  │
                  ▼
S21 ──   MCU ENTERS SLEEP
                  │
                  ▼
S22 ──    MCU RECOVERS
                  │
                  ▼
S23 ──  OUTPUT REQ AND CLK2
                  │
                  ▼
S24 ──   READ OUT DATA
                  │
                  ▼
               ( END )
```

Fig. 10

200

SENSOR

DETECTOR — 12
AS

ADC — 14
CLK1

OSCILLATOR — 13

FREQUENCY CORRECTOR — 16
CON

SD

MEMORY — 15
SD1+SER1
SD2+SER2
...

COMMUNICATION UNIT — 11

REQ
SDi
SERi
CLK2

1A

1

MICROCOMPUTER — 3

COMMUNICATION UNIT — 21

OPERATIONAL CIRCUIT — 23
Ts

CLOCK SIGNAL GENERATOR — 22

CLK2

SDi
SERi

REQ
SDi
SERi
CLK2

300

Fig. 11

Fig. 12

EP 3 416 291 A2

Fig. 13

Fig. 14

Fig. 15

Fig. 16

Fig. 17

**EP 3 416 291 A2**

**Patent documents cited in the description**

- JP 2015228171 A